(19) Europäisches Patentamt
European Patent Office
Office européen des brevets

(11) Publication number: 0 331 493 A2

(12) # EUROPEAN PATENT APPLICATION

(21) Application number: 89302091.7

(22) Date of filing: 02.03.89

(51) Int. Cl.4: A 61 B 19/02
A 61 L 2/26, C 08 G 73/10

(30) Priority: 03.03.88 US 163782

(43) Date of publication of application:
06.09.89 Bulletin 89/36

(84) Designated Contracting States:
BE CH DE FR GB IT LI LU NL

(71) Applicant: AMOCO CORPORATION
200 East Randolph Drive
Chicago Illinois 60601 (US)

(72) Inventor: Sauers, Marvin Edward
Township Line Road, RD No. 1
Belle Mead New Jersey 08502 (US)

Dickinson, Barry Lee
10 Dalley Street
Whitehouse Station New Jersey 08889 (US)

(74) Representative: Laredo, Jack Joseph et al
Elkington and Fife Beacon House 113 Kingsway
London, WC2B 6PP (GB)

(54) **Medical devices made from poly(etherimides).**

(57) Described herein is the use of poly(etherimides) in medical devices where excellent environmental stress-crack resistance is required. The articles made from poly(etherimides) can be steam-sterilized while under stresses of 500 psi or greater; moreover, they are not affected by corrosion-reducing additives such as morpholine, for example; or by typical hospital cleaners and detergents.

EP 0 331 493 A2

## Description

### MEDICAL DEVICES MADE FROM POLY(ETHERIMIDES)

### Field of the Invention

This invention is directed to the use of poly(etherimides) in medical devices where excellent environmental stress-crack resistance is required. The articles made from poly(etherimides) can be steam-sterilized while under stresses of 500 psi or greater; moreover, they are not affected by corrosion-reducing additives such as morpholine, for example. Also, the materials demonstrate good chemical resistance in contact with commonly used hospital cleaners and detergents. The most preferred poly(etherimides) display good transparency, and are based on 2-and/or 3-nitrophthalic anhydrides, bisphenol-A, and metaphenylene diamine.

### Background of the Invention

Poly(aryl ether sulfones) (1) and (2)

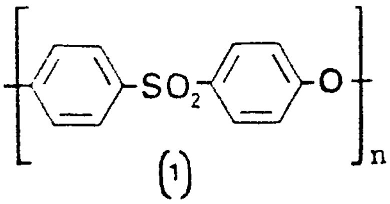

(1)

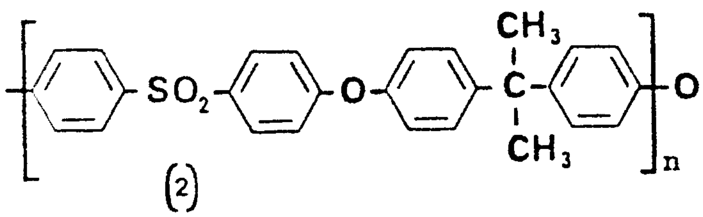

(2)

are commercially available tough thermoplastic materials. They possess a number of attractive features such as excellent high temperature resistance, good electrical properties, and very good hydrolytic stability. Polymer (1) is available from Imperial Chemical Industries, Ltd. under the trademark of Victrex® Poly(ethersulfone). It is produced by the polycondensation of 4,4′-dihydroxydiphenyl sulfone with 4,4′-dichlorodiphenyl sulfone as described in, for example, Canadian patent No. 847,963. The resin contains no aliphatic moieties and has a heat deflection temperature of approximately 210° C. Material (2) is available from Amoco Performance Products, Inc., under the trademark of UDEL. It has a heat deflection temperature of about 180° C, and is made via the nucleophilic polycondensation of bisphenol-A di-sodium salt with 4,4′-dichlorodiphenyl sulfone, as described in U.S. patent No. 4,108,837.

Because of their excellent mechanical and thermal properties, coupled with outstanding hydrolytic stability, the poly(aryl ether sulfones) have been utilized in the medical market for a variety of purposes for at least ten years. These medical devices constitute a wide variety of articles. Obviously, one of the major attributes of the polymers (1) and (2) is their ability to be steam autoclaved repeatedly without loss of properties. Steam autoclaving is a very severe test, requiring both high temperature and hydrolytic stability, and involving cyclical effects - wet/dry, hot/cold.

The poly(aryl ether sulfones) (1) and (2) show some important deficiencies, however. Indeed, parts molded from these materials, stress-crack when steam sterilized under stresses of 500 psi or greater; especially when boiler additives, such as morpholine are employed to reduce corrosion in the steam generating system; or, when in contact with commonly used hospital cleaners and detergents. A way, whereby these deficiencies can be circumvented is described in the present invention.

### The Invention

It was now unexpectedly discovered that poly(etherimides) display superior stress-crack resistance when

steam-sterilized under stresses of 500 psi or greater. The polymers of this class are unaffected by corrosion-reducing additives in the steam generating system (e.g., by morpholine or similar substances); or when in contact with commonly used hospital cleaners and detergents.

The poly(etherimides) are of the formula (3):

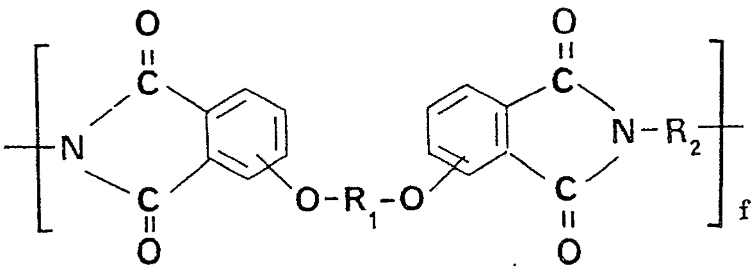

(3)

wherein f is an integer greater than 1, preferably from about 10 to about 10,000 or more; -O-$R_1$-O- is attached to the 3 or 4 and 3′ or 4′ positions and $R_1$ is selected from

(a) a substituted or unsubstituted aromatic radical such as:

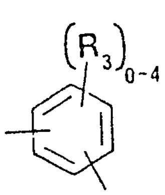

or,

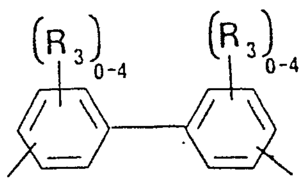

(b) a divalent radical of the formula:

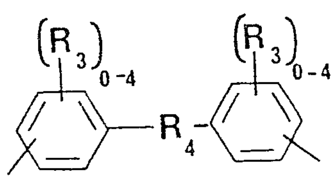

wherein $R_3$ is independently $C_1$ to $C_6$ alkyl, aryl or halogen and $R_4$ is selected from -O-, -S-, $-\overset{\overset{O}{\|}}{C}-$, $-SO_2$, -SO-, alkylene of 1 to 6 carbon atoms, cycloalkylene of 4 to 8 carbon atoms, alkylidene of 1 to 6 carbon atoms, or cycloalkylidene of 4 to 8 carbon atoms; $R_2$ is selected from an aromatic hydrocarbon radical having from 6 to 20 carbon atoms and halogenated derivatives thereof, or alkyl substituted derivatives thereof, wherein the alkyl group contains 1 to 6 carbon atoms, alkylene and cycloalkylene radicals having from 2 to 20 carbon atoms and $C_2$ to $C_8$ alkylene terminated polydiorganosiloxane or a divalent radical of the formulae:

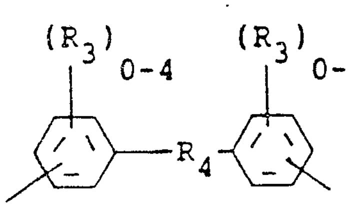

or

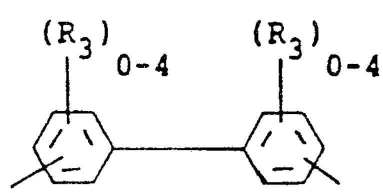

wherein $R_3$ and $R_4$ are as previously defined.

The poly(etherimides) may also be of the following formula:

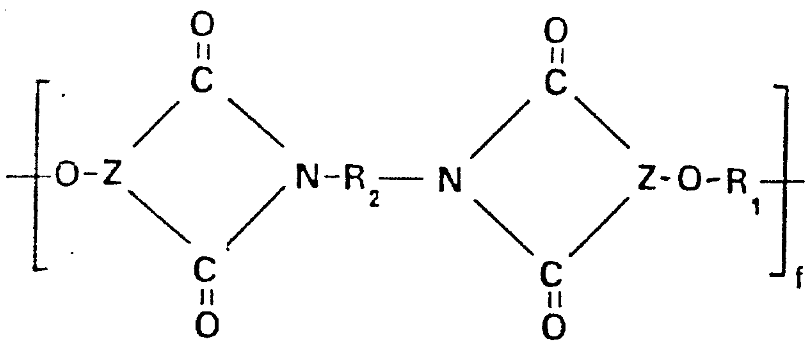

(4)

wherein -O-Z is a member selected from

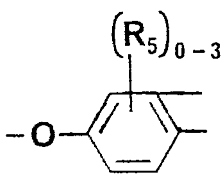

wherein $R_5$ is independently lower alkyl or lower alkoxy

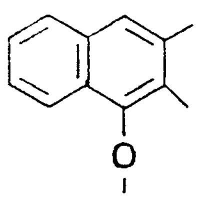

or isomers thereof,

and

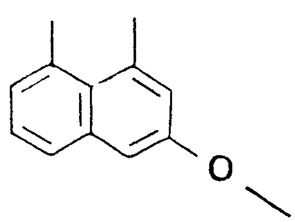

or isomers thereof

wherein the oxygen may be attached to either ring and located ortho or para to one of the bonds of the imide carbonyl groups, and wherein $R_1$ and $R_2$ and f are as previously defined.

These poly(etherimides) are prepared by methods well known in the art as set forth in, for example, U.S. Patent Nos. 3,833,544; 3,887,588; 4,017,511; 3,965,125; and 4,024,110.

The poly(etherimides) of formulae (3) and (4) can, for example, be obtained by any of the methods well known to those skilled in the art including the reaction of any aromatic bis-(ether anhydride) of the formula:

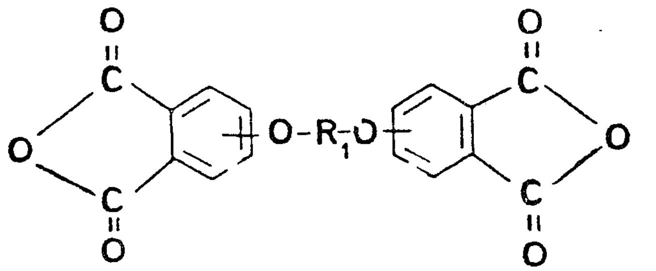

(5)

wherein $R_1$ is as defined hereinbefore, with a diamino compound of the formula

**$H_2N-R_2-NH_2$ (6)**

wherein $R_2$ is as defined hereinbefore. In general, the reactions can be advantageously carried out employing well-known solvents, e.g., o-dichlorobenzene, m-cresol/toluene, N,N-dimethylacetamide, etc., in which the reactions between the dianhydrides and the diamines can be affected, at temperatures of from about 20° to about 250° C. Alternatively, the poly(etherimides) can be prepared by melt polymerization of any dianhydrides of formula (5) with any diamino compound of formula (6) while heating the mixture of the ingredients at elevated temperatures with concurrent intermixing. Generally, melt polymerization temperatures between about 200° to 400° C and preferably 230° to 300° C can be employed. Any order of addition of chain stoppers ordinarily used in melt polymerizations can be employed. The conditions of the reaction and the proportions of ingredients can be varied widely depending on the desired molecular weight, intrinsic viscosity, and types of ingredients used. In general, equimolar amounts of diamine and dianhydride are employed for high molecular weight poly(etherimides); however, in certain instances, a slight molar excess (about 1 to 5 mole percent) of one of the reagents can be employed. Poly(etherimides) of formulae (3) or (4) having an intrinsic viscosity of greater than about 0.2 deciliters per gram, preferably about 0.35 to about 0.60, or about 0.7 deciliters per gram or even higher when measured in m-cresol at 25° C, at a concentration of 0.5 g/100 ml are the most useful.

The aromatic bis(ether anhydride)s of formula (5) include, for example:
2,2-bis[4-(2,3-dicarboxyphenoxy)phenyl]-propane dianhydride;
4,4′-bis(2,3-dicarboxyphenoxy)diphenyl ether dianhydride;
1,3-bis(2,3-dicarboxyphenoxy)benzene dianhydride;
4,4′-bis(2,3-dicarboxyphenoxy)diphenyl sulfide dianhydride;
1,4-bis(2,3-dicarboxyphenoxy)benzene dianhydride;
4,4′-bis(2,3-dicarboxyphenoxy)benzophenone dianhydride;
4,4′-bis(2,3-dicarboxyphenoxy)diphenyl sulfone dianhydride;
2,2′-bis[4-(3,4-dicarboxyphenoxy)phenyl]propane dianhydride;
4,4′-bis(3,4-dicarboxyphenoxy)diphenyl ether dianhydride;
4,4′-bis(3,4-dicarboxyphenoxy)diphenyl sulfide dianhydride;
1,3-bis(3,4-dicarboxyphenoxy)benzene dianhydride;
1,4-bis(3,4-dicarboxyphenoxy)benzene dianhydride;
4,4′-bis(3,4-dicarboxyphenoxy)benzophenone dianhydride;
4,4′-bis(2,3-dicarboxyphenoxy)diphenyl dianhydride;
4,4′-bis(3,4-dicarboxyphenoxy)diphenyl dianhydride;
4-(2,3-dicarboxyphenoxy)-4′-(3,4-dicarboxyphenoxy)diphenyl; etc., and mixtures of such dianhydrides.

The organic diamines of formula (6) include, for example,
m-phenylenediamine, p-phenylenediamine,
2,2-bis(p-aminophenyl)propane,
4,4′-diaminodiphenyl-methane,
4,4′-diaminodiphenyl sulfide,
4,4′-diaminodiphenyl sulfone,
4,4′-diaminodiphenyl ether,
1,5-diaminonaphthalene,
3,3′-dimethylbenzidine, and
3,3′-dimethoxybenzidine; or mixtures of such diamines.

The poly(etherimides) of formulae (3) and (4) may also be prepared by effecting reaction in the presence of an dipolar aprotic solvent of a mixture of ingredients comprising, (a) a bis(nitrophthalimide) of the general formula:

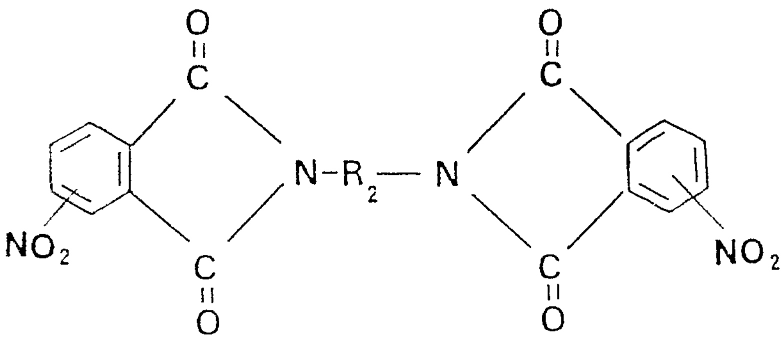

(7)

wherein $R_2$ is defined as hereinabove, and (b) an alkali metal salt of an organic compound of the general formula:

**$MO-R_1-OM$ (8)**

wherein M is an alkali metal and $R_1$ is defined as hereinabove.

The bis(nitrophthalimide) used in preparing the polymer is formed by reacting a diamine of the formula described above, $NH_2-R_2-NH_2$, with a nitro-substituted aromatic anhydride of the formula:

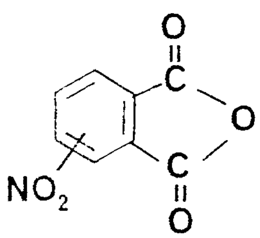

(9)

The molar ratio of diamine to anhydride should ideally be about 1:2 respectively. The initial reaction product is a bis(amide-acid) which is subsequently dehydrated to the corresponding bis(nitrophthalimide).

The diamines are described, supra.

The preferred nitrophthalic anhydrides useful in the present invention are 3-nitrophthalic anhydride, 4-nitrophthalic anhydride and mixtures thereof. These reactants are commercially available in reagent grade. They may also be prepared by the nitration of phthalic anhydride using procedures described in Organic Syntheses, Collective Vol. I, Wiley (1948), page 408. Certain other closely related nitroaromatic anhydrides may also be used in the reaction and are illustrated for example by 2-nitronaphthalic anhydride, 1-nitro-2,3-naphthalene-dicarboxylic anhydride and 3-methoxy-6-nitrophthalic anhydride, and the like.

With reference to the alkali metal salts of formula (8) among the divalent carbocyclic aromatic radicals which $R_1$ may represent (mixtures of such radicals are also included) of particular interest are, for instance, the divalent aromatic hydrocarbon radicals having from 6 to 20 carbon atoms, such as phenylene, biphenylene, naphthylene, etc. Included are residues of, e.g., hydroquinone, resorcinol, chlorohydroquinone, etc. In addition, $R_1$ may be a residue of a dihydroxyl diarylene compound in which the aryl nuclei are joined by either an aliphatic group, a cycloaliphatic group, a sulfoxide group, sulfonyl group, sulfur, carbonyl group, oxygen, a chemical bond, etc. Typical of such diarylene compounds are the following:

2,4-dihydroxydiphenylmethane,
bis(2-hydroxyphenyl)methane,
2,2-bis(4-hydroxyphenyl)propane,
bis(4-hydroxyphenyl)methane,
bis(4-hydroxy-5-nitrophenyl)methane,
bis(4-hydroxy-2,6-dimethyl-3-methoxyphenyl)methane,
1,1-bis(4-hydroxyphenyl)ethane,
1,2-bis(4-hydroxyphenyl)ethane,
1,1-bis(4-hydroxy-2-chlorophenyl)ethane,
1,1-bis(2,5-dimethyl-4-hydroxyphenyl)ethane,

1,3-bis(3-methyl-4-hydroxyphenyl)propane,
2,2-bis(3-phenyl-4-hydroxyphenyl)propane,
2,2-bis(3-isopropyl-4-hydroxyphenyl)propane,
2,2-bis(4-hydroxynaphthyl)propane;
the naphthalene diols; and
bis(4-hydroxyphenyl)ether,
bis(4-hydroxyphenyl)sulfide,
bis(4-hydroxyphenyl)sulfone, and the like.

When dialkali metal salts of formula (8) are used with the compound illustrated by formula (7), the ingredients are advantageously present in an equal molar ratio for optimum molecular weight and properties of the polymer. However, slight molar excesses, e.g., about 0.001 to 0.10 molar excess of either the dinitro-substituted organic compound or of the dialkali metal salt of formula (8) may be employed. When the molar ratios are approximately equal, the polymer is substantially terminated by the end group Z-$NO_2$ at one end and by a phenolic group at the other end. If there is a molar excess of one compound, that particular terminal group will predominate.

The conditions of reaction whereby the alkali-metal salt of formula (8) is reacted with the dinitro-substituted organic compound of formula (7) can be varied widely. Generally, temperatures of the order of about 25 to about 150°C are advantageously employed, although it is possible to employ lower or higher temperature conditions depending on the ingredients used, the reaction product sought, time of reaction, solvent employed, etc. In addition to atmospheric pressure, superatmospheric pressures and subatmospheric pressures may be employed depending upon the other conditions of reaction, the ingredients used, the speed at which it is desired to effect reaction, etc.

The time of reaction also can be varied widely depending on the ingredients used, the temperature, the desired yield, etc. It has been found that times varying from about 5 minutes to as much as 30 to 40 hours are advantageously employed to obtain the maximum yield and desired molecular weight. Thereafter the reaction product can be treated in the appropriate manner required to effect precipitation and/or separation of the desired polymeric reaction product. Generally, common solvents such as alcohols (e.g., methanol, ethanol, isopropyl alcohol, etc.) and aliphatic hydrocarbons (e.g., pentane, hexane, octane, cyclohexane, etc.) may be employed as precipitants for this purpose.

It is important that the reaction between the dinitro-substituted organic compound of formula (7) and the alkali-metal salt of formula (8) (mixtures of such alkali-metal salts can also be used) be carried out in the presence of a dipolar aprotic solvent.

The polymerization is performed under anhydrous conditions usually using dipolar aprotic solvents such as dimethylsulfoxide which are added in varying amounts depending upon the particular polymerization. A total quantity of solvent, dipolar aprotic solvent or mixture of such solvent with an aromatic solvent sufficient to give a final solution containing 10 to 20 percent by weight of polymer is preferably employed.

The preferred poly(etherimides) include those having repeating units of the following formula:

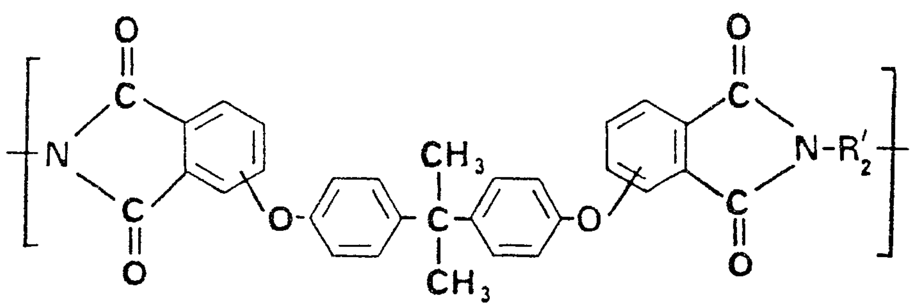

(10)

where $R'_2$ is one or more of

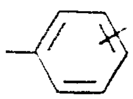 , 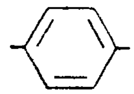 , 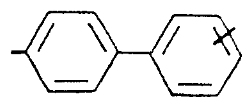 , 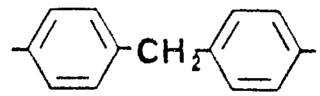 ,

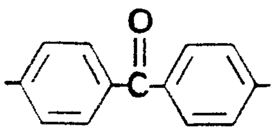 , 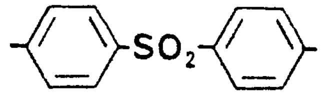 , and 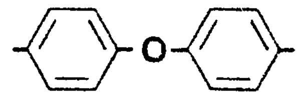

The poly(etherimide) where $R'_2$ is meta-phenylene is most preferred.

The poly(ether-imides) of the instant invention allow for the fabrication of medical devices having outstanding stress-crack resistance. Moreover, articles made from the most preferred poly(etherimides) also display excellent transparency. These devices can be steam-sterilized under stresses of 500 psi or greater and in the presence of a variety of steam boiler additives. Typical boiler additives designed to reduce corrosion in steam generating systems are amino compounds such as morpholine, hydrazine, N,N-diethylaminoethanol ("NALCO 359" or "BETZ NA-9"), and octadecylamine. Steam sterilization is also possible in the presence of various hospital cleaners and detergents, such as those sold under the tradenames of "Castle 7900" (a sonic cleaner), "Chem Crest 14" (an ultrasonic cleaner), "Tergitol Min Foam 2X" (a non ionic surfactant), and the like.

The materials of the instant invention may include pigments, thermal stabilizers, ultraviolet light stabilizers, and other additives.

The instant poly(etherimides) are useful for the fabrication of a wide variety of medical devices. They are of particular interest for autoclavable storage trays such as the systems for storage and delivery of sterile surgical instruments (thus eliminating the costs associated with wrapping); in the medical supply industry for shipment and storage of implants, prostheses and other medical devices under sterile conditions; and in many other similar applications.

## EXAMPLES

The following examples serve to give specific illustration of the practice of this invention but they are not intended in any way to act to limit the scope of this invention.

### Materials

**Polysulfone UDEL P-1700 -**

a poly(aryl ether sulfone) of formula (2), melt-flow at 650°F and 44 psi and 6.5 g/10 minutes. Sold by Amoco Performance Products, Inc.

**Ultem 1000 -**

a poly(etherimide) of formula (10) wherein $R_2$ is meta-phenylene, sold by the General Electric Company.

## Example 1 and Comparative Example A

### Procedure

The materials to be tested were molded into 0.125 inch flex bars (5 inch by 0.5 inch). These bars were autoclaved under constant stress applied via the weighted cantilever beam method. The experiments were performed in a Pelton and Crane tabletop autoclave with a 50 ppm aqueous morpholine solution. Each cycle comprised a steam cycle of 30 minutes at 270°F under 27 psi, followed by a cooling cycle of a minimum of 10 minutes at room temperature. The cooling samples were examined for the presence of any defects, cracks, breakages, etc.

The results, summarized in Table I, clearly show that the poly(etherimides) of the instant invention display steam sterilizability under stresses of 500 psi or greater, in the presence of morpholine. The performance is about twice as good as that of polysulfone UDEL P-1700 under both 1000 and 500 psi stresses. The discrepancies noted in Table I are probably due to slight variations in molding conditions and, hence, quality of the resulting materials.

TABLE I

Steam Sterilization Using a 50 ppm Aqueous Morpholine Solution

| Example | Material | Stress (psi) | Number of Cycles |
|---|---|---|---|
| Comparative A | UDEL P-1700 | 1000 | 79 (fail) |
| | | 500 | 148(fail) |
| | | 0 | 400 |
| 1 | ULTEM 1000 | 1000 | 155 (fail) |
| | | 500 | 289 (fail) |
| | | 0 | 420 |
| | | 1000 | 124 (fail) |
| | | 500 | 290 (fail) |
| | | 0 | 420 |

**Comparative Example B and Example 2**

To determine the effect of cleaners and detergents, a test was run at 180° F for 24 hours using 1/8 inch bars made from UDEL P-1700 (Comparative Example B) and from ULTEM® 1000. Aqueous solutions of various cleaners and detergents were utilized. The stress was applied by bending the bar (constant strain). Note that the stress values were calculated for room temperature; therefore, their actual values may be lower at 180° F. The data are summarized in Table II. The data of Table II clearly show that ULTEM 1000 performs much better than UDEL P-1700.

TABLE II

Effect of Various Cleaners and Detergents

| Reagent | Stress Level (psi) | UDEL P-1700 | ULTEM 1000 |
|---|---|---|---|
| Castle 7900 Sonic Cleaner (2 oz/gal) | 0 | Surface | OK |
| | 1000 | Cracks | OK |
| | 2000 | TD*Cracks | OK |
| | 4000 | Repture Repture | OK |
| Chem Crest 14 Ultrasonic Cleaner (2 oz/gal) | 0 | OK | OK |
| | 1000 | TD*Cracks | OK |
| | 2000 | Rupture | OK |
| | 4000 | Rupture | OK |
| Tergitol Min Foam 2X Monionic Surfactant** (5 ml/liter) | 0 | OK | OK |
| | 1000 | Rupture | OK |
| | 2000 | Rupture | OK |
| | 4000 | Rupture | OK |

* TD = transverse direction.

** Produced by Union Carbide Corporation. The formula is:

$C_{12-14}H_{25-29}O(CH_2CH_2O)_x[CH_2CH_2O/CH_2CH(CH_3)O]_yCH_2CH(CH_3)OH$; Molecular weight is about 640.

## Claims

**1.** A stress-crack resistant medical device steam sterilizable under stresses of 500 psi or greater made from a polyetherimide of the formula

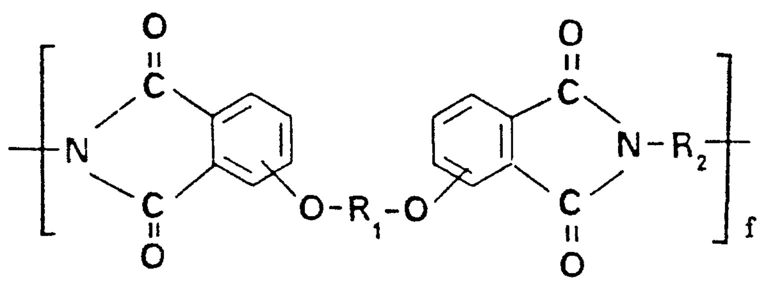

wherein f is an integer greater than 1, preferably from about 10 to about 10,000 or more; $-O-R_1-O-$ is attached to the 3 or 4 and 3′ or 4′ positions and $R_1$ is selected from **(a)** a substituted or unsubstituted aromatic radical such as:

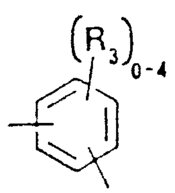

or,

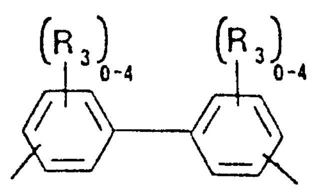

**(b)** a divalent radical of the formula:

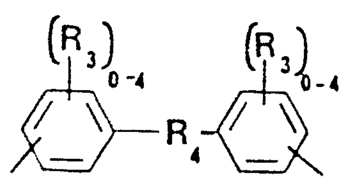

wherein $R_3$ is independently $C_1$ to $C_6$ alkyl, aryl or halogen and $R_4$ is selected from -O-, -S-, $-\overset{\overset{O}{\|}}{C}-$, $-SO_2-$, $-SO-$, alkylene of 1 to 6 carbon atoms, cycloalkylene of 4 to 8 carbon atoms, alkylidene of 1 to 6 carbon atoms, or cycloalkylidene of 4 to 8 carbon atoms; $R_2$ is selected from an aromatic hydrocarbon radical having from 6 to 20 carbon atoms and halogenated derivatives thereof, or alkyl substituted derivatives thereof, wherein the alkyl group contains 1 to 6 carbon atoms, alkylene and cycloalkylene radicals having from 2 to 20 carbon atoms and $C_2$ to $C_8$ alkylene terminated polydiorganosiloxane or a divalent radical of the formulae:

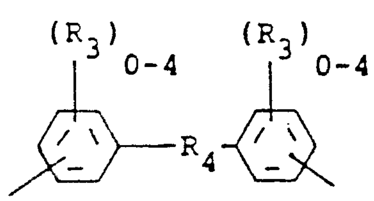

or

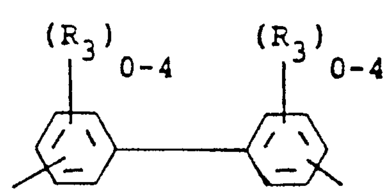

wherein $R_3$ and $R_4$ are as previously defined.

**2.** A stress-crack resistant medical device steam sterilizable under stresses of 500 psi or greater made from a polyetherimide of the formula:

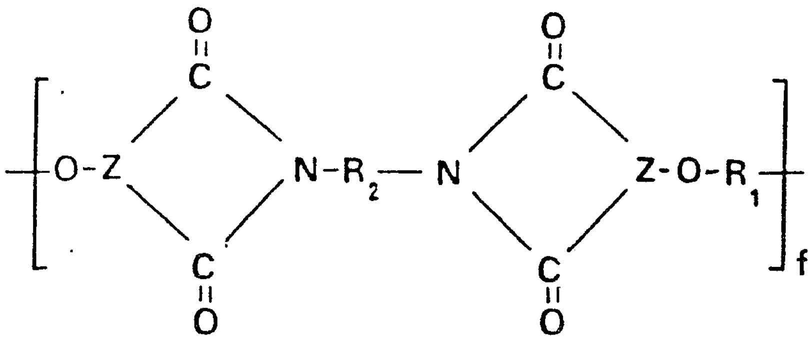

wherein 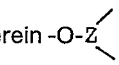 is a member selected from

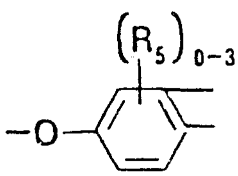

wherein $R_5$ is independently lower alkyl or lower alkoxy

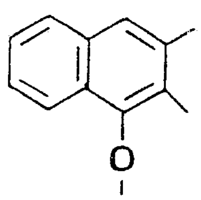 or isomers thereof,

and 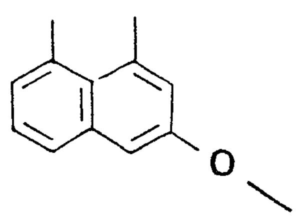

or isomers thereof

wherein the oxygen may be attached to either ring and located ortho or para to one of the bonds of the imide carbonyl groups, and wherein $R_1$, $R_2$ and f are as previously defined.

**3.** A stress-crack resistant medical device steam sterilizable under stresses of 500 psi or greater made from the polyetherimide of formula

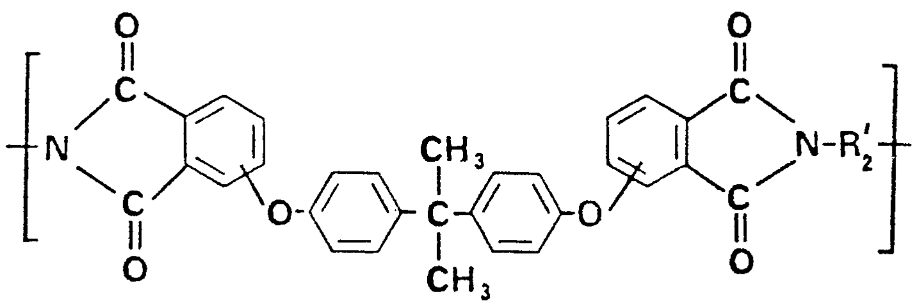

where $R'_2$ is one or more of

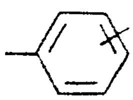 , 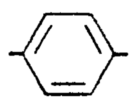 , 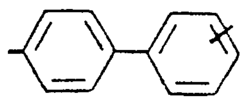 , 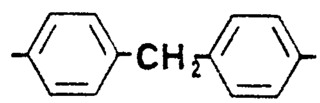 ,

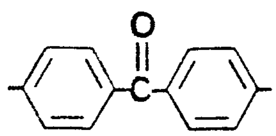 , 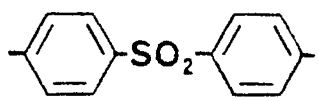 , and 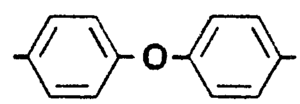

**4.** A medical device as defined in claims **1 or 2 or 3** which is unaffected by corrosion-reducing additives in the steam generating system.

**5.** A medical device as defined in claim **4** where the corrosion-reducing additive is one or more of morpholine, hydrazine, N,N-diethylaminoethanol or octadecylamine.

**6.** A medical device as defined in claim **4** which is resistant to hospital cleaners and detergents.

**7.** A medical device as defined in claim **6** where the cleaner or detergent is selected from the group of sonic cleaners, ultrasonic cleaners and/or non ionic surfactants.

**8.** A medical device as defined in claim **7** where the non ionic surfactant is of the formula

$C_{12-14}H_{25-29}O(CH_2CH_2O)_x[CH_2CH_2O/CH_2CH(CH_3)O]_yCH_2CH(CH_3)OH$

where x and y are one or greater and where the surfactant has a molecular weight of about 640.

**9.** A medical device as defined in claims **1 or 2 or 3** where the poly(etherimide) has a reduced viscosity greater than about 0.2 dl/g, when measured in m-cresol at 25° C, at a concentration of 0.5 g per 100 ml.